# EUROPÄISCHE PATENTSCHRIFT

(11) **EP 3 220 857 B1**
(45) Veröffentlichungstag und Bekanntmachung des Hinweises auf die Patenterteilung: **28.09.2022**
(21) Anmeldenummer: 15825922.6
(22) Anmeldetag: 16.11.2015
(51) Int. Cl.: A61F 2/24

(54) **BIOLOGISCHE HERZKLAPPENPROTHESE**
BIOLOGICAL HEART VALVE PROSTHESIS
PROTHÈSE DE VALVE CARDIAQUE BIOLOGIQUE

(30) Priorität: 18.11.2014 DE 102014223522
(43) Veröffentlichungstag der Anmeldung: 27.09.2017
(73) Patentinhaber: Sievers, Hans-Hinrich, 24119 Kronshagen (DE)
(72) Erfinder: SCHARFSCHWERDT, Michael, 23564 Lübeck (DE); HOF, Andreas, 23568 Lübeck (DE); SIEVERS, Hans-Hinrich, 24119 Kronshagen (DE)
(74) Vertreter: Patentanwälte Vollmann Hemmer Lindfeld Partnerschaft mbB
(86) Internationale Anmeldenummer: PCT/DE2015/200502
(87) Internationale Veröffentlichungsnummer: WO 2016/078659

(56) Entgegenhaltungen:
- WO-A1-95/28899
- WO-A1-03/068108

## Beschreibung

Die Erfindung betrifft eine Herzklappenprothese mit den im Oberbegriff des Anspruchs 1 angegebenen Merkmalen.

Beispielsweise aus DE 10 2010 051 632 B4 ist eine biologische Herzklappenprothese bekannt, welche ein Klappengestell aufweist, an welchem mehrere Klappensegel aus biologischem Material befestigt sind. Am Axialende des Klappengestells ist ein Nahtring aus einem Gewebematerial angeordnet, um die Herzklappenprothese in die Aorta einnähen zu können. Beim Einsetzen solcher Herzklappenprothesen ist es problematisch, eine Herzklappenprothese in einer für den jeweiligen Patienten passenden Größe zu finden, sodass sie sich möglichst ohne Verengung des Strömungsquerschnittes in die Aorta einsetzen lässt.

WO 95/28899 A1 offenbart eine gestentete biologische Herzklappenprothese, welche ein Klappengestell mit daran befestigten Klappensegeln aufweist. Am Rand des Klappengestells ist ein Befestigungsring zum Einnähen der Herzklappenprothese ausgebildet, welcher in seinem Inneren einen Tragring sowie ein Polster zum Vernähen aufweist. Das Polster mit dem Tragring schließt sich direkt an die Bögen des Klappengestells an. Insofern besteht das Problem, dass bei erforderlichen Verformungen dieses Befestigungsbereiches auch das Klappengestell beim Einsetzen verformt wird, sodass es zu einer Beeinträchtigung der Funktionalität der Klappensegel kommen kann.

Ein ähnliches Problem stellt sich bei dem aus WO 03/068108 A1 bekannten Stand der Technik, welcher eine ähnliche Herzklappenprothese offenbart. Diese weist einen Tragring im Befestigungsbereich auf, welcher im Bereich der Bögen des Klappengestells mit diesem verbunden ist. Die Befestigung im Bereich der Bögen hat jedoch den Nachteil, dass in diesem Bereich Kräfte direkt auf das Klappengestell übertragen werden.

Vor dem Hintergrund dieses Standes der Technik ist es Aufgabe der Erfindung, eine Herzklappenprothese bereitzustellen, welche sich einfacher in ein Blutgefäß einsetzen lässt und dabei zuverlässig den gewünschten Strömungsquerschnitt und eine zuverlässige Funktion der Klappensegel sicherstellt.

Diese Aufgabe wird durch eine Herzklappenprothese mit den in Anspruch 1 angegebenen Merkmalen gelöst. Bevorzugte Ausführungsformen ergeben sich aus den Unteransprüchen, der nachfolgenden Beschreibung sowie den beigefügten Figuren.

Bei der erfindungsgemäßen Herzklappenprothese handelt es sich insbesondere um eine Aortenklappenprothese.

Die erfindungsgemäße Herzklappenprothese weist ein Klappengestell auf, an welchem mehrere Klappensegel bzw. Taschenklappen, vorzugsweise in bekannter Weise drei Klappensegel befestigt sind. Das Klappengestell besteht vorzugsweise aus einem gebogenen metallischen Draht, welcher so geformt ist, dass drei Bögen gebildet sind, welche mit ihren Enden aneinander angrenzen und über kleinere Verbindungsbögen miteinander verbunden sind. Diese Verbindungsbögen bilden Kommissuren zwischen den Klappensegeln, welche in den Bögen befestigt sind. Die Verbindungsbögen, das heißt die Spitzen der Kommissuren bilden ein erstes, insbesondere stromabwärtiges Axialende der Herzklappenprothese. Am entgegengesetzten zweiten Axialende der Herzklappenprothese befindet sich ein axial an das Klappengestell anschließender Befestigungsbereich, welcher zur Befestigung, insbesondere zum Vernähen in einem Blutgefäß, z. B. der Aorta ausgebildet ist. In diesem Bereich kann beispielsweise ein aus einem elastischen Material oder einem Gewebe bzw. Textil gebildeter Nahtring angeordnet sein, durch welchen hindurch die Herzklappenprothese mit dem umgebenden Gefäß vernäht werden kann.

Erfindungsgemäß ist in dem Befestigungsbereich ein Stabilisierungsring angeordnet. Dieser Stabilisierungsring weist eine vorgegebene Form, insbesondere eine Form mit kreisförmigem Querschnitt auf. Der Stabilisierungsring definiert so einen vorgegebenen Durchmesser des Befestigungsbereiches und ist erfindungsgemäß kraftentkoppelt mit dem Klappengestell verbunden. Das heißt die Verbindung zwischen Stabilisierungsring und Klappengestell ist elastisch bzw. beweglich ausgebildet, sodass auf den Stabilisierungsring wirkende Kräfte nicht oder im Wesentlichen nicht auf das Klappengestell übertragen werden und umgekehrt. So ist eine flexible Verbindung zwischen dem Stabilisierungsring und dem Klappengestell vorhanden, welche eine gewisse Beweglichkeit und Kraftentkopplung sicherstellt. Die kraftentkoppelte Verbindung zwischen dem Stabilisierungsring und dem

Klappengestell hat den Vorteil, dass die auf den Befestigungsbereich wirkenden Kräfte nicht auf das Klappengestell übertragen werden, sodass das Klappengestell durch die Befestigung nicht verformt und mit Kraft beaufschlagt wird, sodass die Funktionalität der Klappensegel nicht beeinträchtigt wird. Dies ermöglicht es, die Herzklappenprothese wesentlich strammer und passgenauer in ein Gefäß einzusetzen, da durch den Stabilisierungsring gewisse Kräfte auf das umgebende Gewebe ausgeübt werden können und gegebenenfalls auch eine gewisse Aufweitung des umgebenden Gewebes erreicht werden kann. So wird es möglich, eine maximal große Herzklappenprothese in das Gefäß einzusetzen, sodass der Strömungsquerschnitt gegenüber dem natürlichen Querschnitt im Wesentlichen nicht eingeschränkt wird. Gleichzeitig wird jedoch sichergestellt, dass bei diesem passgenauen strammen Einsetzen der Herzklappenprothese es nicht zu einer unerwünschten Verformung oder Bewegungsbeeinträchtigung des Klappengestells und der Klappensegel kommt.

Die Klappensegel sind vorzugsweise aus biologischem Material gefertigt. Die Klappensegel können in bekannter Weise, beispielsweise durch Vernähen mit dem Klappengestell verbunden sein. Das Klappengestell ist dabei weiter bevorzugt in bekannter Weise von einer Umhüllung, insbesondere aus einem Textil- bzw. Gewebematerial umgeben. Die Umhüllung verschließt dabei insbesondere den Bereich der oben beschriebenen Kommissuren zwischen den die Klappensegel tragenden Bögen des Klappengestells.

Das Klappengestell ist bevorzugt elastisch verformbar ausgebildet. So verbessert sich die Beweglichkeit der Klappensegel. Darüber hinaus kann das Klappengestell derart elastisch verformbar, insbesondere in radialer Richtung elastisch verformbar sein, dass es zum Einsetzen der Herzklappenprothese in ein Gefäß in seinem Querschnitt bzw. Durchmesser verkleinert werden kann und sich nach dem Einsetzen aufweiten kann, sodass es sich nach dem Einsetzen in seinem Durchmesser in die Sinus aortae hinein erweitern kann. Das Klappengestell kann insbesondere vor dem Einsetzen in radialer Richtung elastisch verformt werden, um den Außenquerschnitt zu verkleinern. Durch geeignete Haltemittel kann das Klappengestell beim Einsetzen in dieser verformten Stellung gehalten werden und dann nach dem Einsetzen durch Lösen der Haltemittel elastisch wieder in die entspannte Ursprungsform zurück verformt werden. Dabei kann sich das Klappengestell dann in radialer Richtung aufweiten. In einem entspannten Zustand weist das Klappengestell vorzugsweise einen größeren Außenquerschnitt als der Stabilisierungsring auf. So kann sich das Klappengestell in die Sinus aortae hinein erweitern. So kann ein möglichst großer Strömungsquerschnitt sichergestellt werden. Eine solche Ausgestaltung ist beispielsweise aus DE 10 2010 051 632 B4 bekannt, auf welche an dieser Stelle verwiesen wird.

Besonders bevorzugt ist der Stabilisierungsring mit dem Klappengestell über ein Textil- bzw. Gewebematerial verbunden, wobei das Textil- oder Gewebematerial vorzugsweise das Klappengestell und/oder den Stabilisierungsring umhüllt. Ein solches Textil- oder Gewebematerial stellt die ausreichend bewegliche Verbindung zwischen Klappengestell und Stabilisierungsring her, sodass eine Kraftentkopplung gegeben ist. Ferner kann das Textil- bzw. Gewebematerial gleichzeitig dazu genutzt werden, die Herzklappenprothese mit dem umgebenden Blutgefäß, in welches die Herzklappenprothese eingesetzt wird, zu vernähen.

Der Stabilisierungsring weist zumindest in radialer Richtung eine geringere elastische Verformbarkeit als das Klappengestell auf und ist vorzugsweise im Wesentlichen starr ausgebildet. Wenn der Stabilisierungsring in radialer Richtung eine geringere elastische Verformbarkeit als das Klappengestell aufweist, kann er vorzugsweise relativ zu dem Klappengestell als starr angesehen werden. Während das Klappengestell verformbar ist, um sich beispielsweise, wie oben beschrieben, nach dem Einsetzen aufzuweiten und die erforderliche Beweglichkeit der Klappensegel sicherzustellen, ist der Stabilisierungsring vorzugsweise derart starr, dass er nach dem Einsetzen durch das umgebende Gefäß im Wesentlichen nicht verformt wird und vielmehr besonders bevorzugt in der Lage ist, das umgebende Gefäß auf den von dem Stabilisierungsring vorgegebenen Durchmesser zu dehnen. So wird durch den Stabilisierungsring ein definierter Querschnitt, das heißt Strömungsquerschnitt der Herzklappenprothese sichergestellt.

Der Stabilisierungsring ist vorzugsweise aus Metall, das heißt insbesondere einem körperverträglichen Material gefertigt. Besonders bevorzugt kann der Stabilisierungsring aus einer Formgedächtnislegierung, wie Nitinol, gefertigt sein. Die Ausgestaltung aus einer Formgedächtnislegierung hat den Vorteil, dass der Stabilisierungsring vor dem Einsetzen in das Gefäß verformt, insbesondere in seinem Durchmesser verkleinert werden kann, um das Einsetzen zu vereinfachen. Anschließend kann durch Temperaturänderung der Stabilisierungsring wieder in seine ursprüngliche definierte Form aufgeweitet werden, in welcher er die oben beschriebene Stabilisierungsfunktion übernimmt. Wenn er die vordefinierte Form wieder angenommen hat, ist er vorzugsweise ausreichend starr, das heißt in der oben beschriebenen Weise so wenig elastisch, dass er durch das umgebende Gewebe des Gefäßes nicht oder im Wesentlichen nicht verformt wird und so einen definierten Querschnitt sicherstellt.

Der Stabilisierungsring weist in axialer Richtung auskragende, fest mit dem Stabilisierungsring verbundene Kommissurenstützen aufweisen, welche sich als Vorsprünge in die von dem Klappengestell gebildeten Kommissuren erstrecken. Das heißt, diese Vorsprünge erstrecken sich in den Bereich zwischen den Bögen des Klappengestells, welche oben beschrieben wurden und dienen dazu, die Kommissuren zu stützen und insbesondere das die Kommissuren verschließende Material, insbesondere Textil- oder Gewebematerial zu stützen, sodass es im Bereich der Kommissuren durch dieses Material bzw. diese Umhüllung des Klappengestells nicht zu einer Einschränkung des Strömungsquerschnittes kommt. So kann auch bei elastischer Verformbarkeit des Klappengestells in diesem Bereich eine definierte Form der Kommissuren durch die festen Vorsprünge sichergestellt werden.

Weiter bevorzugt definiert der Stabilisierungsring einen Innenquerschnitt, welcher dem von den Kommissuren des Klappengestells begrenzten Innenquerschnitt entspricht. So wird vorzugsweise von dem Stabilisierungsring gemeinsam mit den Kommissuren der Innenquerschnitt der Herzklappenprothese definiert.

Der Stabilisierungsring weist bevorzugt einen Innenquerschnitt auf, welcher dem Querschnitt des natürlichen Blutgefäßes, insbesondere der Aorta, entspricht, für welche die Herzklappenprothese dimensioniert ist. Das heißt, beim Einsetzen wird eine für das Blutgefäß passende Herzklappenprothese ausgewählt, welche einen Stabilisierungsring hat, dessen Innenquerschnitt dem Durchmesser bzw. Querschnitt des natürlichen Blutgefäßes entspricht. So kann, wie beschrieben, beim Einsetzen das Blutgefäß um ein gewisses Maß aufgeweitet bzw. gedehnt werden, sodass nach dem Einsetzen der Herzklappenprothese deren Innenquerschnitt, insbesondere der Innenquerschnitt im Bereich des Stabilisierungsrings, dem natürlichen Innenquerschnitt des Blutgefäßes entspricht, sodass es zu keiner Verengung des Strömungsweges kommt.

Gemäß einer besonderen Ausführungsform der Erfindung kann der Stabilisierungsring an seinem Außenumfang mit Auskragungen, das heißt insbesondere radial nach außen gerichteten Auskragungen versehen sein, welche zum Eingriff in umgebendes Gewebe, d. h. Körpergewebe vorgesehen und insbesondere in Form eines Gewindes ausgebildet sind. Diese Auskragungen dienen der Fixierung des Stabilisierungsringes im Blutgefäß. Diese Fixierung kann zusätzlich zu einem Vernähen oder besonders bevorzugt alternativ zum Vernähen genutzt werden. So wird eine nahtlose (sutureless) Befestigung möglich. Wenn die Auskragungen in Form eines oder mehrerer Gewindegänge ausgebildet sind oder einzelne Auskragungen so angeordnet sind, dass sie zusammen eine Gewindeform definieren, wird es möglich, die Herzklappenprothese mit dem Stabilisierungsring in das Blutgefäß einzuschrauben. Besonders bevorzugt sind Auskragungen vorgesehen, welche Hinterschneidungen bilden bzw. in Form von Widerhaken ausgebildet sind, sodass eine sichere, gegen Lösen gesicherte Fixierung erreicht wird. Die Auskragungen bzw. Vorsprünge greifen in das Gewebe ein und halten bzw. Fixieren den Stabilisierungsring und damit die gesamte Herzklappenprothese.

Die Anordnungen von Auskragungen bzw. Vorsprüngen zum Eingriff in das umgebende Gewebe ist nicht auf die vorangehend beschriebene und in dieser Anmeldung beanspruchte Herzklappenprothese und insbesondere nicht auf eine biologische Herzklappenprothese beschränkt. Eine solche Ausgestaltung kann bei jeder Herzklappenprothese zur Anwendung kommen. Z.B. kann auch eine mechanische Herzklappenprothese mit einem so ausgestalteten Befestigungsring bzw. Stabilisierungsring mit Auskragungen versehen sein. So ist Gegenstand dieser Anmeldung auch eine Herzklappenprothese, welche einen Befestigungsring aufweist, der an seinem Außenumfang mit Auskragungen bzw. Vorsprüngen versehen ist, welche zum Eingriff in umgebendes Gewebe vorgesehen sind und insbesondere in Form eines Gewindes ausgebildet sind. Ein solcher Befestigungsring würde hinsichtlich seiner Befestigung dem vorangehend und nachfolgend beschriebenen Stabilisierungsring entsprechen.

Gemäß einer weiteren bevorzugten Ausführungsform sind die Auskragungen verformbar oder aus einem Formgedächtnismaterial wie Nitinol ausgebildet. Dies ermöglicht es, die Auskragungen in ihrer Ausrichtung zwischen zumindest zwei Positionen zu verändern. So können zum Einsetzen der Herzklappenprothesen die Auskragungen sich in einer ersten Position befinden, in welcher sie nicht radial nach außen auskragen, so dass die Herzklappenprothese ohne Beschädigung des Gewebes in dieses eingesetzt werden kann. In einer zweiten Position können die Auskragungen dann so gerichtet sein, dass sie radial nach außen gerichtet sind und in das Gewebe eingreifen und so die Herzklappenprothese im Gewebe sichern. Dabei ist zu verstehen, dass gegebenenfalls auch nur ein Teil der Auskragungen oder Abschnitte der Auskragungen in dieser Weise zwischen einer ersten Position und einer zweiten Position beweg- bzw. verformbar sein können. Beispielsweise können Abschnitte oder einzelne Auskragungen so angeordnet sein, dass sie in ihrer zweiten Position Widerhaken bilden und in ihrer ersten Position so gelegen sind, dass sie ein Einsetzen der Herzklappenprothese, insbesondere ein Einschrauben nicht behindern. Die Bewegung bzw. Verformung zwischen der ersten und zweiten Position kann bei Verwendung eines Formgedächtnismaterials beispielsweise durch Erwärmung erfolgen. Jedoch sind auch andere Methoden zur Verformung möglich, beispielsweise auch die Aufweitung mit Hilfe eines Ballons, durch welchen die Auskragungen nach außen in ihre zweite Position gedrückt werden.

Gemäß einer weiteren bevorzugten Ausgestaltung sind die Auskragungen an dem Stabilisierungsring bzw. Befestigungsring im Bereich der Kommissuren bzw. der interleaflet triangles frei von Auskragungen. Das sind diejenigen Bereiche der Herzklappenprothese, welche im eingesetzten Zustand der Herzklappenprothese den genannten interleaflet triangles gegenüberliegen. Hierdurch wird verhindert, dass in diesen Bereichen die Auskragungen das umliegende Gewebe und insbesondere die dort gelegenen Nerven verletzen. Dies sind insbesondere die Bereiche, an welchen die Kommissurenstützen der Herzklappenprothese, wie sie vorangehend beschrieben wurden, gelegen sind.

Der Stabilisierungsring ist mit radialen Durchbrechungen, insbesondere sich radial erstreckenden Durchgangslöchern versehen. Diese können zum einen dazu dienen, Fäden zum Vernähen durch den Stabilisierungsring hindurchzuführen. Zum anderen können die Durchbrechungen auch dazu dienen, dass Gewebe des Blutgefäßes in diese Durchbrechungen eindringen kann, und so zur Fixierung des Stabilisierungsringes im Blutgefäß beitragen.

Weiter bevorzugt kann die erfindungsgemäße Herzklappenprothese einen flexiblen Nahtring in dem Befestigungsbereich aufweisen, mit welchem die Herzklappenprothese im Blutgefäß vernäht werden kann. Dabei ist besonders bevorzugt ein flexibler Nahtring vorgesehen, welcher den Stabilisierungsring zumindest abschnittsweise in dessen Au-βenumfang überlappt. Dies ermöglicht es, das Gewebe des Blutgefä-βes, an welchem die Herzklappenprothese vernäht werden soll, zwischen diesem flexiblen Nahtring und dem Stabilisierungsring anzuordnen und durch den Stabilisierungsring und den flexiblen Nahtring hindurch zu vernähen. Alternativ kann in dem Befestigungsbereich an dem Stabilisierungsring ein zweiter Nahtring vorgesehen sein, welcher von dem am Außenumfang überlappenden Nahtring überlappt wird, sodass das Gewebe zwischen zwei Nahtringen fixiert und vernäht werden kann. Bei dieser Ausgestaltung kann auf die beim Vernähen sonst üblichen Filze verzichtet werden, da das Gewebe so zwischen zwei Nahtringen oder zwischen dem Stabilisierungsring und dem Nahtring fixiert wird, wobei die Nahtringe und gegebenenfalls der Stabilisierungsring als Gegenlager für die beim Vernähen verwendeten Faden dienen.

Erfindungsgemäß bilden der Stabilisierungsring mit dem Klappengestell und den Klappensegeln eine vorgefertigte Baueinheit, welche dafür vorgesehen ist, als Ganzes in ein Blutgefäß eingesetzt zu werden. Das heißt, es ist nicht erforderlich, beim Einsetzen oder nach dem Einsetzen eine Verbindung zwischen Stabilisierungsring und Klappengestell herzustellen.

Gemäß einer weiteren bevorzugten Ausführungsform der Erfindung, welche auch unabhängig von den vorangehend beschriebenen Ausgestaltungen realisiert werden kann, weist die Herzklappenprothese ein Klappengestell mit mehren, vorzugsweise drei Klappensegeln auf. Diese Klappensegel sind bevorzugt aus biologischem Material gefertigt. Die Klappensegel weisen gemäß dieser bevorzugten Ausführungsform eine derartige Form auf, dass sie im geöffneten Zustand der Herzklappenprothese Abschnitte der Umfangsfläche eines Zylinders, insbesondere eines Kreiszylinders definieren. Im geöffneten Zustand spannen die Klappensegel somit gemeinsam mit den von dem Klappengestell gebildeten Kommissuren im Wesentlichen eine kreiszylindrische Umfangsfläche auf, welche einen maximal möglichen Strömungsquerschnitt ermöglicht.

Um dies zu ermöglichen, sind die Klappensegel bevorzugt so geformt, dass sie an jeder axialen Position zwischen zwei gegenüberliegenden Anknüpfungspunkten an das Klappengestell die Form eines Kreisbogens beschreiben, wobei alle Kreisbögen denselben Krümmungsradius aufweisen. Das heißt die vom Klappensegel in Umfangsrichtung verlaufenden Verbindungslinien zweier gegenüberliegender Punkte an einem Bogen des Klappengestells haben die Form eines Kreisbogens, wobei an jeder axialen Position des Klappensegels diese Kreisbögen denselben Krümmungsradius aufweisen. So wird gewährleistet, dass die Klappensegel sich derart weit öffnen können, dass sie gemeinsam im Wesentlichen die Kontur eines Kreiszylinders aufspannen und so einen maximalen Strömungsquerschnitt freigeben.

Diese Form der Klappensegel wird besonders bevorzugt durch eine spezielle Form der Außenkontur des Klappensegels, welche an das Klappengestell angrenzt, erreicht. Jedes Klappensegel hat zunächst einmal grundsätzlich zwei Bereiche der Außenkontur. Ein erster Bereich bildet die freien Stirnkanten bzw. Anlagekanten des Klappensegels, welche im geschlossenen Zustand mit den Stirnkanten der anderen Klappensegel dichtend in Anlage kommen. Ein zweiter Bereich grenzt an einen Bogen des Klappengestells an und ist dort mit dem Klappengestell verbunden. Dieser Bereich der Außenkontur, welcher an das Klappengestell angrenzt, das heißt an einem Bogen des Klappengestells befestigt ist, weist eine Form bzw. Kontur auf, welche im Wesentlichen aus zumindest drei, vorzugsweise fünf Kreisbögen, das heißt Abschnitten von Kreisen, zusammengesetzt ist. Die Kontur weist zunächst zwei äußere Kreisbögen an einander entgegengesetzten Seiten auf, welche vorzugsweise denselben Krümmungsradius aufweisen und nach außen gekrümmt sind. Das heißt die Mittelpunkte, um welche diese Kreisbögen gekrümmt sind, liegen außerhalb der Außenkontur des Klappensegels. Diese äußeren Kreisbögen grenzen an die freien Stirnkanten, welche dichtend mit den freien Stirnkanten der anderen Klappensegel in Anlage kommen, an. Zwischen diesen beiden äußeren Kreisbögen ist zumindest ein innerer Kreisbogen gelegen, welcher nach innen gekrümmt ist. Das heißt der Mittelpunkt, um welchen dieser Kreisbogen gekrümmt ist, liegt im Inneren der Außenkontur des Klappensegels bzw. der von dieser Außenkontur aufgespannten Fläche. Besonders bevorzugt sind drei innere Kreisbögen vorgesehen, wobei ein mittlerer der inneren Kreisbögen einen größeren Krümmungsradius hat als die zwei seitlich des mittleren inneren Kreisbogens gelegenen Kreisbögen, welche ihrerseits an die äußeren Kreisbögen angrenzen. Diese zwei an die äußeren Kreisbögen angrenzenden inneren Kreisbögen haben vorzugsweise denselben Krümmungsradius, sind jedoch um verschiedene Punkte gekrümmt. Insgesamt hat die Außenkontur des Klappensegels, welche an das Klappengestell angrenzt, wenn das Klappensegel flach ausgebreitet ist somit eine im Wesentlichen U-förmige Form, wobei die freien Enden der Schenkel dieses U nach außen, das heißt voneinander weg gekrümmt sind.

Es ist zu verstehen, dass die beschriebene spezielle Formgebung der Klappensegel unabhängig von der Verwendung eines Stabilisierungsoder Befestigungsringes, wie er vorangehend beschrieben wurde, verwirklicht werden kann. Insofern ist die erfindungsgemäße Ausgestaltung der Form der Klappensegel nicht auf die Kombination mit diesen Merkmalen beschränkt.

Nachfolgend wird die Erfindung beispielhaft anhand der beigefügten Figuren beschrieben. In diesen zeigt:
- Fig. 1: eine perspektivische Ansicht einer erfindungsgemäßen Herzklappenprothese,
- Fig. 2: eine Draufsicht auf die Herzklappenprothese gemäß Fig. 1 von unten,
- Fig. 3: eine perspektivische Ansicht des Klappengestells und des Stabilisierungsringes der Herzklappenprothese gemäß Fig. 1 und 2,
- Fig. 4: eine Seitenansicht des Klappengestells und des Stabilisierungsringes gemäß Fig. 3,
- Fig. 5: eine Draufsicht auf die Anordnung des Klappengestells und des Stabilisierungsringes gemäß Fig. 4 von oben,
- Fig. 6: eine Seitenansicht eines Stabilisierungsringes gemäß einer zweiten Ausführungsform der Erfindung,
- Fig. 7: eine Schnittansicht des Stabilisierungsringes gemäß Fig. 7,
- Fig. 8: eine Draufsicht auf den Stabilisierungsring gemäß Fig. 6,
- Fig. 9: eine perspektivische Ansicht eines Stabilisierungsringes gemäß einer dritten Ausführungsform der Erfindung,
- Fig. 10: eine Schnittansicht des Stabilisierungsringes gemäß Fig. 9,
- Fig. 11: eine perspektivische Ansicht eines Stabilisierungsringes gemäß einer vierten Ausführungsform der Erfindung,
- Fig. 12: eine Schnittansicht des Stabilisierungsringes gemäß Fig. 11,
- Fig. 13: eine perspektivische Ansicht eines Stabilisierungsringes gemäß einer fünften Ausführungsform der Erfindung,
- Fig. 14: eine Seitenansicht des Stabilisierungsringes gemäß Fig. 13,
- Fig. 15: eine perspektivische Darstellung des Anheftungsrandes für die Klappensegel,
- Fig. 16: die Konstruktion des Außenrandes einen Klappensegels und
- Fig. 17: eine schematische Darstellung der Anheftungspunkte und Länge (Segelbreite) eines Klappensegels bei seiner maximalen Öffnung.

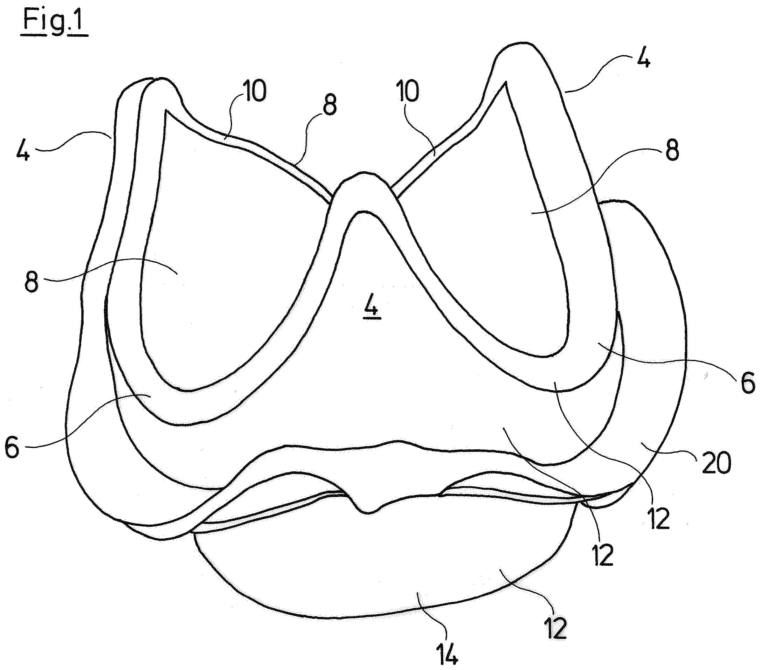

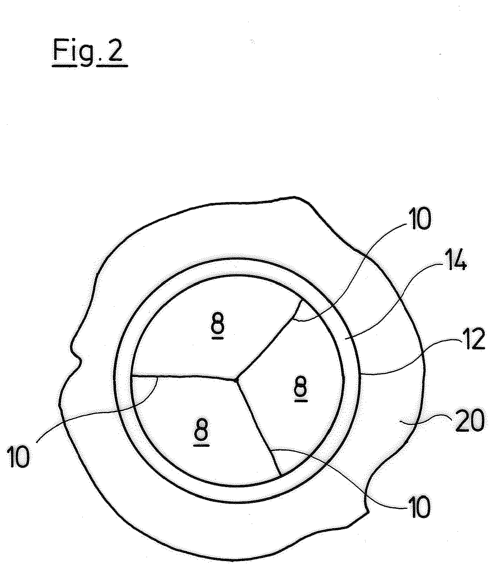

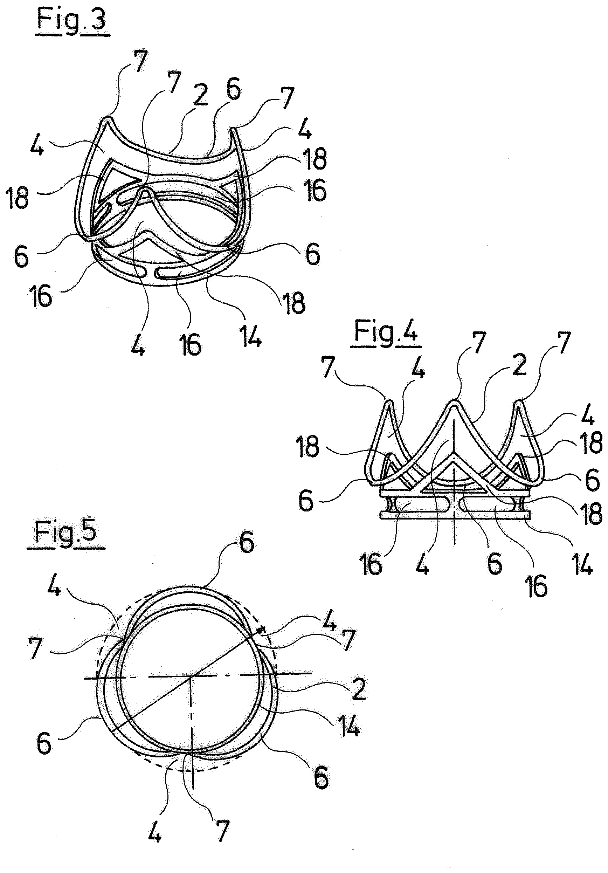

Die erfindungsgemäße Herzklappenprothese, welche in Fig. 1 und 2 gezeigt ist, weist in ihrem Inneren, wie in den Fig. 3 bis 5 gezeigt ist, ein Klappengestell 2 auf, welches drei Bögen 6 aufweist, welche an ihren Enden durch Verbindungsbögen 7 miteinander verbunden sind und so drei Kommissuren 4 bilden. Das Klappengestell 2 ist aus einem metallischen Draht geformt, welcher elastisch verformbar ist. Die drei Bögen 6 tragen drei Klappensegel 8 aus biologischem Material. Diese Taschenklappen bzw. Klappensegel 8 stoßen an drei Anlagekanten 10 aneinander und dichten dort gegeneinander ab. Zum Öffnen bewegen sich die Klappensegel 8 radial nach außen, sodass sie an den Anlagekanten 10 außer Anlage treten und eine zentrale Öffnung freigeben.

Das Klappengestell 2 ist im Übrigen von einer Umhüllung 12 aus einem Textil- bzw. Gewebematerial umhüllt, welches sich auch über die Kommissuren 4 erstreckt und diese umfänglich verschließt. Ein erstes stromabwärtiges Axialende der Herzklappenprothese wird von den Spitzen bzw. Verbindungsbögen 7 der Kommissuren 4 definiert. Am entgegengesetzten stromaufwärtigen Axialende ist ein Befestigungsbereich ausgebildet. Dort befindet sich im Inneren der Umhüllung 12 ein Stabilisierungsring 14. Der Stabilisierungsring 14 ist aus Metall gefertigt und weist eine definierte Kreisringform auf. Der Innenquerschnitt des Stabilisierungsringes 14 entspricht im Wesentlichen dem Innenquerschnitt zwischen den Spitzen der Kommissuren 14 und definiert den maximalen Strömungsquerschnitt durch die Herzklappenprothese. Dies ist insbesondere in Fig. 5 zu erkennen.

Gegenüber dem Klappengestell 2 ist der Stabilisierungsring 14 starr ausgebildet, das heißt er weist eine geringere Elastizität bzw. Verformbarkeit als das Klappengestell 2 auf. Insbesondere ist der Stabilisierungsring 14 in radialer Richtung im Wesentlichen nicht elastisch verformbar. Der Stabilisierungsring 14 weist in radialer Richtung Durchbrechungen 16 auf. Diese Durchbrechungen 16 ermöglichen es, Nahtmaterial bzw. Fäden durch den Stabilisierungsring 14 zu führen, um die Herzklappenprothese an dem Stabilisierungsring 14 in einem Blutgefäß zu befestigen. Ferner weist der Stabilisierungsring 14 an einem Axialende drei sich in die Kommissuren 4 des Klappengestells 2 hinein erstreckende Kommissurenstützen 18 auf. Diese Kommissurenstützen 18 stützen im Bereich der Kommissuren 4 die Umhüllung 12, sodass diese in definierter Form gehalten wird.

Wie in den Fig. 3 und 4, welche die Anordnung des Klappengestells 2 und des Stabilisierungsringes 14 im Inneren der in Fig. 1 gezeigten Herzklappenprothese darstellen, zu erkennen ist, sind das Klappengestell 2 und der Stabilisierungsring 14 nicht direkt miteinander verbunden. Das Klappengestell 2 und der Stabilisierungsring 14 werden vielmehr allein durch die flexible Umhüllung 12 aus Textil- bzw. Gewebematerial miteinander verbunden, sodass eine kraftentkoppelte Verbindung zwischen dem Klappengestell 2 und dem Stabilisierungsring 14 gegeben ist. Aufgrund der Flexibilität des Gewebematerials der Umhüllung 12 kann sich das Klappengestell 2 relativ zu dem Stabilisierungsring 14 bewegen. Dies ermöglicht eine elastische Verformbarkeit des Klappengestells 2, insbesondere in radialer Richtung, während der Stabilisierungsring 14 aufgrund seiner starren Ausgestaltung nicht verformt wird. Durch die Umhüllung 12 bilden der Stabilisierungsring 14 und das Klappengestell 2 mit den darin ausgebildeten Klappensegeln 8 dennoch eine vorgefertigte Baueinheit, welche als Ganzes in ein Blutgefäß eingesetzt werden kann, ohne dass bei der Operation noch Montagetätigkeiten oder ein Vernähen zwischen Stabilisierungsring 14 und dem Klappengestell 2 mit dem Klappensegel 8 erforderlich wäre.

Die Fig. 3 bis 5 zeigen den unverformten entspannten Zustand des Klappengestells 2. Es ist zu erkennen, dass sich die Bögen 6 des Klappengestells 2 in diesem Zustand radial über den Außenumfang des Stabilisierungsrings 14 hinaus erstrecken, während die Kommissuren 4 im Wesentlichen auf dem Umfang des Stabilisierungsringes 14 gelegen sind. So können sich die Bögen 6 des Klappengestells 2 im implantierten Zustand in die Sinus aortae hinein erstrecken und so einen maximalen Innenquerschnitt freigeben, welcher im Wesentlichen dem natürlichen Querschnitt der Aorta entspricht. Dazu wird die gezeigte Herzklappenprothese in ihrer Dimension so auf das jeweilige Blutgefäß abgestimmt, dass der Innendurchmesser des Stabilisierungsrings 14 im Wesentlichen dem natürlichen Innendurchmesser der Aorta entspricht oder geringfügig größer ist. So kann eine gewisse Aufweitung des Gewebes des Blutgefäßes beim Einsetzen erreicht werden, sodass insgesamt der Strömungsquerschnitt durch diese Herzklappenprothese nicht eingeschränkt wird.

Zum Einsetzen kann das Klappengestell 2, wie aus DE 10 2010 051 622 B4 bekannt ist, radial nach innen bewegt werden, sodass die Bögen 6 radial nach innen verformt werden und der maximale Außendurchmesser durch den Stabilisierungsring 14 definiert wird. So wird das Vernähen erleichtert. Nach dem Vernähen kann diese Verformung des Klappengestells 2 gelöst werden, sodass die Bögen 6 sich dann, wie vorangehend beschrieben, aufgrund ihrer Elastizität wieder erweitern und insbesondere in die Sinus aortae hinein erweitern können. Dies ist insbesondere aufgrund der flexiblen, kraftentkoppelten Anbindung des Klappengestells 2 an dem Stabilisierungsring 14 möglich. Der Stabilisierungsring 14 ist ausreichend starr ausgebildet, um den Innenquerschnitt der Herzklappenprothese zu definieren und fest in das Blutgefäß, das heißt die Aorta eingesetzt und dort fixiert zu werden. Dabei gibt der Stabilisierungsring 14 eine definierte Größe und Form der Herzklappenprothese und insbesondere deren Strömungsquerschnitts vor.

Der Stabilisierungsring 14 kann aus einer Formgedächtnislegierung, wie Nitinol, gefertigt sein, sodass es möglich ist, ihn vor dem Einsetzen radial so zu verformen, dass sein Außenquerschnitt verkleinert wird. Nach dem Einsetzen kann er sich dann durch Temperaturänderung in die gezeigte Ausgangsform zurück bewegen und dann fest in einem Blutgefäß fixiert werden, wo er dann als starre Struktur den gewünschten Querschnitt definiert.

An der Umhüllung 12 ist ferner am Außenumfang ein Nahtring 20 aus Textil- bzw. Gewebematerial angeordnet, welcher den Stabilisierungsring 14 außenumfänglich überlappt. Dies ermöglicht es, die Herzklappenprothese so in das Blutgefäß einzunähen, dass das Gewebe des Blutgefäßes zwischen dem Stabilisierungsring 14 und dem Nahtring 20 zu liegen kommt und die Fäden zum Vernähen durch den Nahtring 20, das zwischenliegende Körpergewebe und dann durch den Stabilisierungsring 14 geführt werden können. Dabei wird das Nahtmaterial durch die Umhüllung, welche den Stabilisierungsring 14 umgibt und die Durchbrechungen 16 in dem Stabilisierungsring 14 geführt. Der Stabilisierungsring 14 bildet somit ein Gegenlager beim Vernähen, sodass zusätzliche Filze als Gegenlager nicht erforderlich sind.

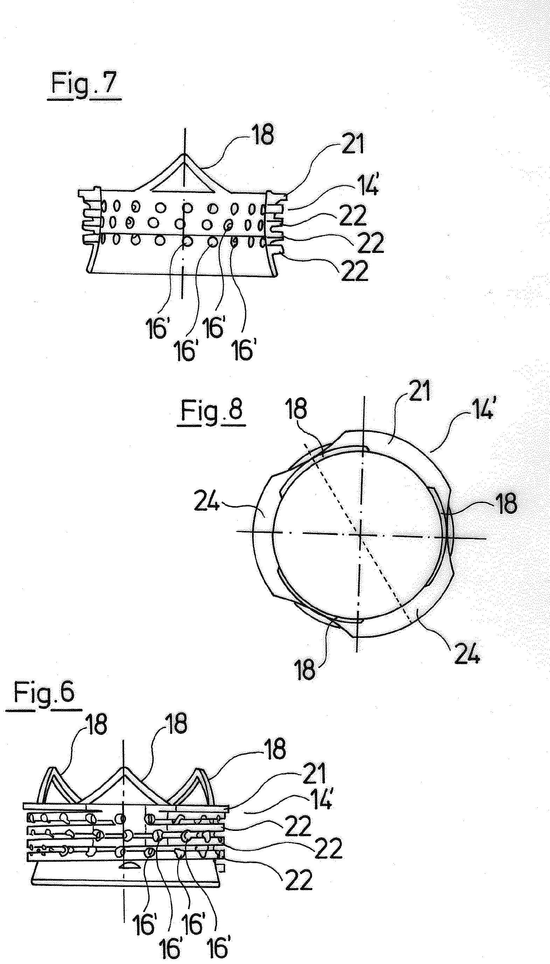

In Fig. 6 bis 8 ist ein alternativer Stabilisierungsring 14' gezeigt, welcher anstelle des vorangehend beschriebenen Stabilisierungsrings 14 verwendet werden kann. Im Unterschied zu dem vorangehend beschriebenen Stabilisierungsring 14 weist der alternative Stabilisierungsring 14' an seinem Außenumfang Gewindegänge 22 auf, mit deren Hilfe der Stabilisierungsring 14' mit den daran angebrachten übrigen Teilen der Herzklappenprothese in das Blutgefäß eingedreht bzw. eingeschraubt werden kann. Die Gewindegänge 22 greifen dabei in das Körpergewebe ein und bilden eine feste Fixierung des Stabilisierungsringes 14' und damit der gesamten Herzklappenprothese in dem Blutgefäß. Die Durchbrechungen 16' am Stabilisierungsring 14' sind als kreisförmige Durchgangslöcher, welche sich in radialer Richtung erstrecken, ausgebildet. Diese Durchbrechungen 16' können zum einen ebenfalls dazu dienen, zur zusätzlichen Fixierung Nahtmaterial durch den Stabilisierungsring 14' zu führen. Zum anderen bilden sie an den Gewindegängen 22 Hinterschneidungen, welche zu einem derart formschlüssigen Eingriff zwischen dem Körpergewebe und den Gewindegängen 2 führen, dass die Drehbewegung nach dem Einsetzen blockiert wird und ein fester Eingriff erreicht wird. Vorzugsweise wird eine Herzklappenprothese mit dem Stabilisierungsring 14' so ohne zusätzliches Vernähen im Blutgefäß fixiert.

Die übrigen Teile einer Herzklappenprothese mit dem Stabilisierungsring 14' können in der vorangehend beschriebenen Weise ausgebildet sein. Auch die Kommissurenstützen 18 sind in identischer Weise zu der vorangehenden Beschreibung ausgebildet. An demjenigen Axialende, an welchem die Kommissurenstützen 18 ausgebildet sind und welches dem Klappengestell 2 zugewandt ist, weist der Stabilisierungsring 14' einen radial nach außen gerichteten Kragen 21 auf, welcher der Befestigung an der Umhüllung 12 bzw. einem Gewebematerial dient, welches den Stabilisierungsring 14' mit dem Klappengestell 2 und den Klappensegeln 8 verbindet. Das heißt, bei dieser Ausführungsform kann der Stabilisierungsring 14' unumhüllt bleiben, sodass er direkt mit dem Körpergewebe in Eingriff tritt.

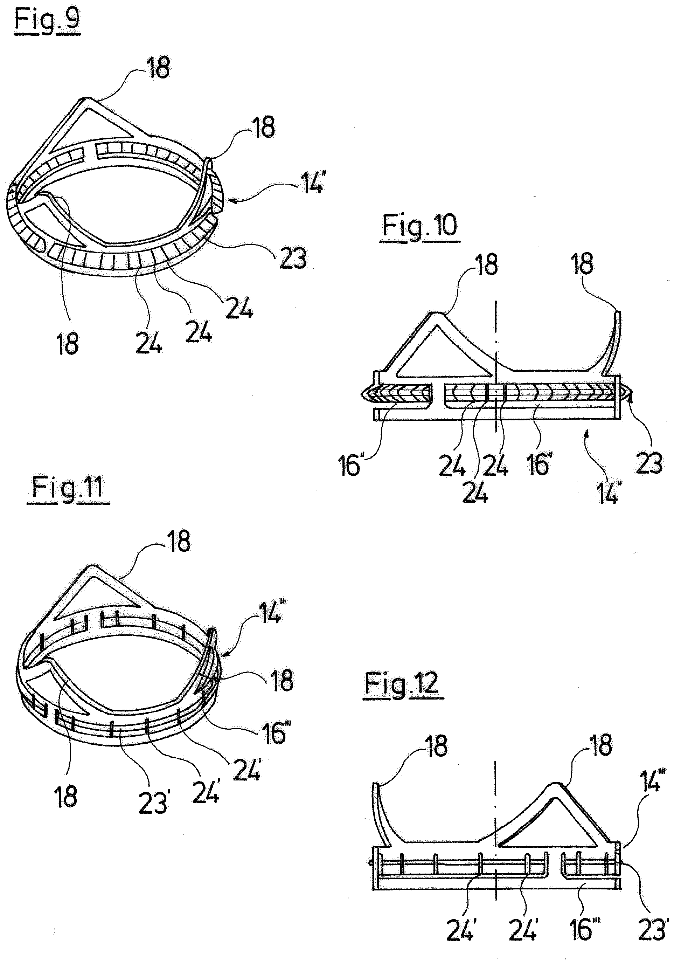

Die Figuren 9 und 10 zeigen eine dritte Variante eines Stabilisierungsringes 14". Anstelle der Gewindegänge 22 bei der vorangehend beschriebenen Ausführungsform, weist der Stabilisierungsring 14" der dritten Ausführungsform anders gestaltete Vorsprünge bzw. Auskragungen 23 auf, welche radial nach außen von dem Stabilisierungsring 14" auskragen bzw. vorstehen. In diesem Ausführungsbeispiel werden die Auskragungen von einer im Querschnitt v-förmigen, im Wesentlichen sich über den ganzen Umfang erstreckenden, Ausbuchtung gebildet. Dieser Ausbuchtung weist eine Vielzahl von Einschnitten 24 auf, so dass eine Vielzahl nebeneinander liegender im Querschnitt v-förmiger Auskragungen bzw. Zähne gebildet wird, wobei die Spitzen der Auskragungen jeweils den radial außenliegenden Punkt bilden. Diese Auskragung bzw. Zähne können in das umliegende Gewebe zur Fixierung des Stabilisierungsringes 14" eingreifen. Die Auskragungen sind mit einem Axialende fest mit dem Stabilisierungsring 14" verbunden. Am entgegengesetzten Axialende ist eine bogenförmige Durchbrechung 16" vorhanden. Diese kann, wie oben beschrieben, zum einen genutzt werden, um Nahtmaterial durch den Stabilisierungsring 14" hindurchzuführen. Zum anderen ist die Durchbrechung 16" vorzugsweise so ausgebildet, dass die Auskragungen 23 aus ihrer gewinkelten Form radial nach innen verformt werden können, das sie im Wesentlichen nicht über den Außenumfang des Stabilisierungsringes 14" nach außen vorkragen. So kann eine Einbauposition geschaffen werden, in welcher der Stabilisierungsring 14' in die Aorta eingesetzt werden kann. Nach dem Einsetzen können die Auskragungen 23 dann in die gezeigte auskragende Position verschwenkt bzw. verformt werden, um mit dem umgebenden Gewebe zur Fixierung des Stabilisierungsringes 14' in Eingriff zu treten. Dies kann beispielsweise durch mechanischen Druck von innen, beispielsweise mit Hilfe eines Ballons oder durch Verwendung eines Formgedächtnismaterials geschehen. Wir ein Formgedächtnismaterial wie Nitinol verwendet, können die Auskragungen 23 durch Erwärmung in die in den Figuren 9 und 10 gezeigte Ruhelage zurückverformt werden. Hinsichtlich der übrigen Ausgestaltungen des Stabilisierungsringes in Figuren 9 und 10 wird auf die vorangehende Beschreibung der ersten beiden Ausführungsformen verwiesen.

Der in Figuren 11 und 12 gezeigte Stabilisierungsring 14‴ unterscheidet sich von der Ausgestaltung gemäß Figuren 9 und 10 in der Weise, dass die die Auskragungen 23 bildende radial nach außen gerichtete, umlaufende Ausbuchtung im Querschnitt kleiner ausgebildet ist und die Einschnitte 24' weiter beabstandet sind. So sind die einzelnen Elemente, Zähne bzw. Auskragungen 23' größer, wobei die radialen Vorsprünge in radialer Richtung kürzer ausgebildet sind. Im Übrigen entspricht die Funktion der vorangehenden Beschreibung. Auch die Durchbrechung 16'" kann zum selben Zweck genutzt werden, wir die vorangehend beschriebene Durchbrechung 16". Hinsichtlich der übrigen Ausgestaltung des Stabilisierungsringes 14‴ wird auf die vorangehende Beschreibung der ersten drei Ausführungsformen verwiesen.

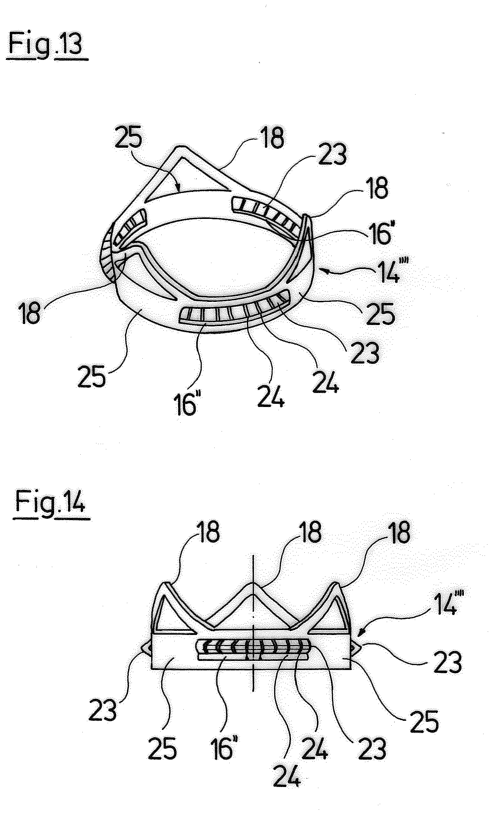

Die Figuren 13 und 14 zeigen einen Stabilisierungsring 14ʺʺ, welcher eine Variante der Ausführungsform in Figuren 9 und 10 darstellt, der Unterschied in der Ausgestaltung des Stabilsierungsringes 14ʺʺ zu der Ausgestaltung des Stabilisierungsringes 14" besteht darin, dass die Umfangsabschnitte 25 des Stabilisierungsringes 14‴, welche im Bereich der Kommissurenstützen 18 gelegen sind, frei von Auskragungen 23 sind. Die Außenumfangsabschnitte 25 des Stabilisierungsringes 14ʺʺ sind die Umfangsbereiche, welche im eingebauten Zustand der Herzklappenprothese an den interleaflet triangles in der Aorta anliegen. Diese Bereiche sind frei von Auskragungen 23, um in diesen Bereichen eine Beschädigung des Gewebes zu vermeiden. Bezüglich der Ausführungsform in Figuren 13 und 14 ist zu verstehen, dass auch die vorangehend beschriebenen Ausführungsformen des Stabilisierungsringes gemäß Figuren 6 - 12 in dieser Weise ausgestaltet werden können, dass die Umfangsabschnitte 25 im Bereich der Kommissurenstützen 18, welche den interleaflet trianlges beim Einbau gegenüberliegen, frei von Auskragungen sind.

Im Übrigen ist hinsichtlich sämtlicher Ausführungsformen gemäß Figuren 6 - 14 zu verstehen, dass die dort beschriebene Befestigung mit Hilfe von Auskragungen im Gewebe zur Unterstützung oder zum Ersatz eines Vernähens (sutureless Befestigung) auch bei anderen Herzklappenprothesen, insbesondere mechanischen Herzklappenprothesen zum Einsatz kommen kann.

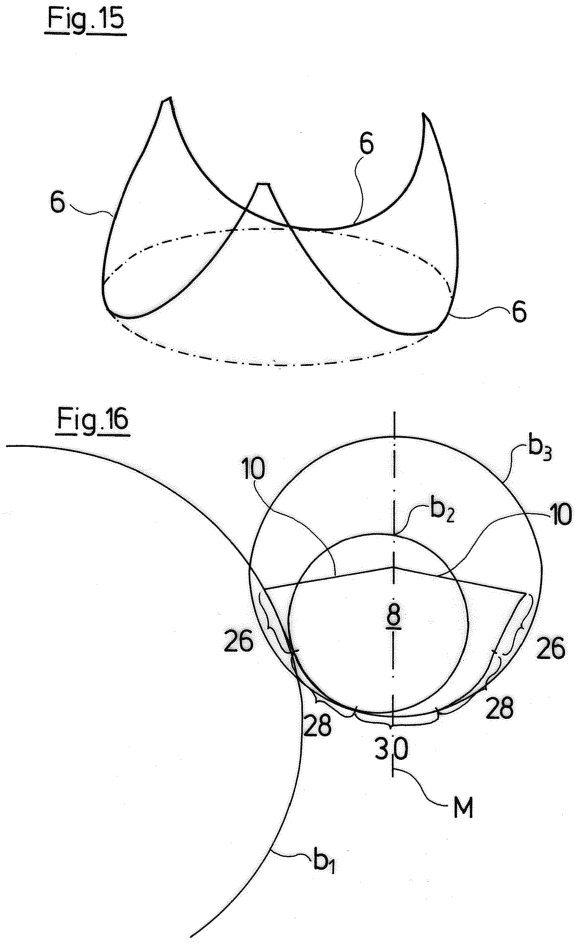

Für die in Figuren 1 - 5 gezeigte Herzklappenprothese wird nun nachfolgend anhand der Figuren 15 - 17 eine spezielle Form der Klappensegel beschrieben. Diese Form der Klappensegel könnte auch unabhängig von der Verwendung eines Befestigungs- bzw. Stabilisierungsringes 14, 14'. 14", 14‴ und 14ʺʺ Verwendung finden.

Fig. 15 zeigt schematisch die drei Bögen 6 des Klappengestelles, welche gleichzeitig den Anheftungsrand der Klappensegel 8 definieren. Die Klappensegel 8 weisen, wenn sie flach ausgebreitet sind, vorzugsweise eine Form auf, wie sie in Fig. 16 gezeigt sind. Ein erster Bereich der Außenkontur bildet die freien Stirnkanten, welche die Anlagekanten 10 definieren, mit welchen das Klappensegel 8 an den zwei anderen Klappensegeln dichtend zur Anlage kommt. Ein zweiter Bereich der Au-βenkontur bildet den Anheftungsrand an einem Bogen 6 des Klappengestelles 2. Dieser Anheftungsrand ist aus fünf Kreisabschnitten bzw. Kreisbögen gebildet. Die beiden äußeren Kreisbögen 26, welche an die Anlagekanten 10 angrenzen, haben denselben Krümmungsradius, jeweils eines Kreises bzw. Kreisbogens b₁. Diese beiden Kreisbögen 26 sind nach außen gebogen, das heißt die Mittelpunkte der Kreise b₁ liegen außerhalb der Fläche des Klappensegels 8. Das Klappensegel 8 hat daher eine Form, welche sich zu den Anlagekanten 10 im Bereich der äußeren Kreisbögen 26 zunehmend aufweitet. Nach innen anschließend an die äußeren Kreisbögen 26 sind erste innere Kreisbögen 28 gelegen, welche ebenfalls identisch gekrümmt sind und einen Krümmungsradius aufweisen, welcher jeweils einem zweiten Kreis b₂ entspricht. Der Krümmungsradius der zweiten Kreise b₂ ist kleiner als der Krümmungsradius des Kreises b₁. Die ersten inneren Kreisbögen 28 sind nach innen gekrümmt. Das heißt die Mittelpunkte, um welche die beiden Kreisbögen 28 gekrümmt sind, liegen innerhalb der Kontur des Klappensegels 8. Zwischen den beiden ersten inneren Kreisbögen 28 ist ein zweiter innerer Kreisbogen 30 gelegen, welcher ebenfalls nach innen gekrümmt ist und einen Krümmungsradius aufweist, welcher einem dritten Kreis b₃ entspricht, wobei dieser dritte Kreis b₃ einen Radius aufweist, welcher größer als der Radius der Kreise b₂ und kleiner als der Radius der Kreise b₁ ist.

Insgesamt ist das Klappensegel 8 symmetrisch zu einer Mittellinie M ausgebildet, wobei der Mittelpunkt des Kreises b₃ auf dieser Mittellinie M liegt. Die Mittellinie M erstreckt sich ferner durch die Mitte des zweiten inneren Kreisbogens 30. Ferner liegt der Berührungspunkt der beiden Anlagekanten 10 auf dieser Mittel- bzw. Symmetrielinie M. Ausgehend von dem Schnittpunkt der Mittellinie M mit dem zweiten inneren Kreisbogen 30 weist die Außenkontur des Klappensegels 8, welche die Anheftungslinie an dem Bogen 6 definiert, somit eine zunächst im Übergang von dem zweiten inneren Kreisbogen 30 zu dem ersten inneren Kreisbogen 28 zunehmende nach innen gerichtete Krümmung auf, um dann im Übergang von dem ersten inneren Kreisbogen 28 zu dem äußeren Kreisbogen 26 in eine nach außen gerichtete geringere Krümmung überzugehen.

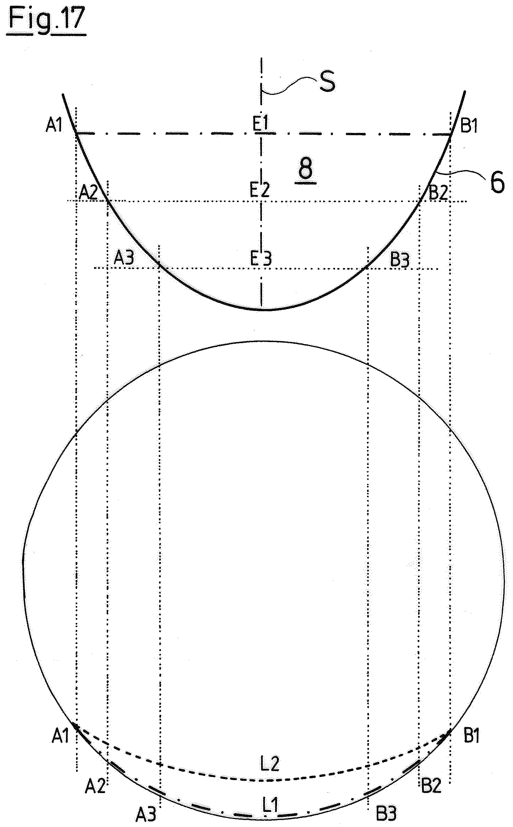

Durch diese Gestaltung der Klappensegel wird erreicht, dass die Klappensegel sich so öffnen können, dass sie insgesamt im Wesentlichen Abschnitte der Außenumfangsfläche eines Kreiszylinders definieren, das heißt eine kreisförmige Öffnung von maximal großem Querschnitt freigeben. Dies wird anhand von Fig. 17 erläutert. Dort werden beispielhaft drei Anheftungspunkte A₁, A₂ und A₃ und entsprechend in Ebenen E1, E2 und E3 jeweils gegenüberliegende Anheftungspunkte B₁, B₂ und B₃ betrachtet. Dabei ist der Anheftungspunkt B₁ spiegelsymmetrisch zu dem Anheftungspunkt A₁, der Anheftungspunkt B₂ spiegelsymmetrisch zu dem Anheftungspunkt A₂ und der Anheftungspunkt B₃ spiegelsymmetrisch zu dem Anheftungspunkt A₃ bezüglich einer Symmetrielinie S des Bogens 6. Wenn das Klappensegel 8 in seiner geöffneten Position ist, liegen alle drei Verbindungslinien, das heißt die Verbindungslinie zwischen den Anheftungspunkten A₁ und B₁, die Verbindungslinie zwischen den Anheftungspunkte A₂ und B₂ und die Verbindungslinie zwischen den Anheftungspunkten A₃ und B₃ auf demselben Kreisbogen L1, welcher die kreisförmige Öffnung definiert. Dies würde auch für alle anderen Linien normal zu der Symmetrielinie S zwischen den beiden Seiten des Bogens 6, das heißt zwischen zwei gegenüberliegenden Anheftungspunkten gelten. So kann sich das Klappensegel 8 in geöffnetem Zustand in die Form eines Umfangsabschnittes eines Kreiszylinders mit dem Querschnitt des Kreisbogens L1 bewegen und so eine maximale Öffnung sicherstellen. In Fig. 17 ist im Unterschied hierzu schematisch gestrichelt die Öffnungslinie L2 herkömmlicher Klappensegel gezeigt, welche sich dadurch ergibt, dass die Länge des Klappensegels zwischen den Anheftungspunkten A₁ und B₁ nicht ausreichend lang ist.

Es ist zu verstehen, dass die Ausgestaltung der Klappensegel 8, wie sie anhand der Figuren 15 bis 17 erläutert wurde, auch unabhängig von der Verwendung eines Stabilisierungsringes 14, 14', 14", 14‴ und 14ʺʺ, wie er vorangehend beschrieben wurde, Verwendung finden kann.

### Bezugszeichenliste

- 2: - Klappengestell
- 4: - Kommissuren
- 6: - Bögen
- 7: - Verbindungsbögen
- 8: - Klappensegel
- 10: - Anlagekante
- 12: - Umhüllung
- 14, 14', 14", 14‴, 14ʺʺ: - Stabilisierungsring
- 16, 16': - Durchbrechungen
- 18: - Kommissurenstützen
- 20: - Nahtring
- 21: - Kragen
- 22: - Gewindegänge
- 23, 23': - Auskragungen
- 24, 24': - Einschnitte
- 25: - Umfangsabschnitte
- 26: - äußere Kreisbögen
- 28: - 1. Innere Kreisbögen
- 30: - 2. Innerer Kreisbogen
- b₁, b₂, b₃: - Kreise
- M: - Mittellinie
- S: - Symmetrielinie
- L2: - Öffnungslinie
- A₁, A₂, A₃, B₁, B₂, B₃: - Anheftungspunkte
- E1, E2, E3: - Ebenen

## Patentansprüche

1. Herzklappenprothese mit einem Klappengestell (2), an welchem mehrere Klappensegel (8) befestigt sind, sowie einem sich axial an das Klappengestell (2) anschließenden Befestigungsbereich zur Befestigung in einem Blutgefäß,
**dadurch gekennzeichnet, dass**
der Befestigungsbereich einen Stabilisierungsring (14; 14', 14", 14‴, 14ʺʺ) aufweist, welcher eine vorgegebene Form und einen vorgegebenen Durchmesser des Befestigungsbereiches definiert, der Stabilisierungsring (14; 14', 14", 14‴, 14ʺʺ) in axialer Richtung auskragende, fest mit dem Stabilisierungsring (14; 14', 14", 14‴, 14ʺʺ) verbundene Kommissurenstützen (18) aufweist, welche sich in die von dem Klappengestell (2) gebildeten Kommissuren (4) erstrecken,
der Stabilisierungsring (14; 14', 14", 14‴, 14ʺʺ) mit radialen Durchbrechungen (16; 16')versehen ist,
der Stabilisierungsring (14; 14', 14", 14‴, 14ʺʺ) zumindest in radialer Richtung eine geringere elastische Verformbarkeit als das Klappengestell (2) aufweist,
und kraftentkoppelt über eine flexible Verbindung derart mit dem Klappengestell (2) verbunden ist, dass die auf den Befestigungsbereich wirkenden Kräfte nicht auf das Klappengestell übertragen werden und das Klappengestell durch die Befestigung nicht verformt und mit Kraft beaufschlagt wird.

2. Herzklappenprothese nach Anspruch 1, **dadurch gekennzeichnet, dass** die Klappensegel (8) aus biologischem Material gefertigt sind.

3. Herzklappenprothese nach Anspruch 1 oder 2, **dadurch gekennzeichnet, dass** das Klappengestell (2) elastisch verformbar ausgebildet ist.

4. Herzklappenprothese nach einem der vorangehenden Ansprüche, **dadurch gekennzeichnet, dass** der Stabilisierungsring (14; 14', 14", 14‴, 14ʺʺ) mit dem Klappengestell (2) über ein Gewebematerial (12) verbunden, wobei das Gewebematerial vorzugsweise das Klappengestell (2) und/oder den Stabilisierungsring (14; 14', 14", 14‴, 14ʺʺ) umhüllt.

5. Herzklappenprothese nach einem der vorangehenden Ansprüche, **dadurch gekennzeichnet, dass** der Stabilisierungsring (14; 14', 14", 14‴, 14ʺʺ) aus Metall und insbesondere aus einer Formgedächtnislegierung ausgebildet.

6. Herzklappenprothese nach einem der vorangehenden Ansprüche, **dadurch gekennzeichnet, dass** der Stabilisierungsring (14; 14', 14", 14‴, 14ʺʺ) einen Innenquerschnitt definiert, welcher dem von den Kommissuren (4) des Klappengestells (2) begrenzten Innenquerschnitt entspricht.

7. Herzklappenprothese nach einem der vorangehenden Ansprüche, **dadurch gekennzeichnet, dass** das Klappengestell (2) in radialer Richtung elastisch verformbar ist.

8. Herzklappenprothese nach einem der vorangehenden Ansprüche, **dadurch gekennzeichnet, dass** das Klappengestell (2) in einem entspannten Zustand einen größeren Außenquerschnitt als der Stabilisierungsring (14; 14', 14", 14‴, 14ʺʺ) aufweist.

9. Herzklappenprothese nach einem der vorangehenden Ansprüche, **dadurch gekennzeichnet, dass** der Stabilisierungsring (14; 14', 14", 14‴, 14ʺʺ) einen Innenquerschnitt aufweist, welcher dem Querschnitt des natürlichen Blutgefäßes, insbesondere der Aorta, entspricht, für welche die Herzklappenprothese dimensioniert ist.

10. Herzklappenprothese nach einem der vorangehenden Ansprüche, **dadurch gekennzeichnet, dass** der Stabilisierungsring (14') an seinem Außenumfang mit Auskragungen (22) versehen ist, welche zum Eingriff in umgebendes Gewebe vorgesehen und insbesondere in Form eines Gewindes (22) ausgebildet sind.

11. Herzklappenprothese nach einem der vorangehenden Ansprüche, **gekennzeichnet durch** einen flexiblen Nahtring (20), welcher den Stabilisierungsring (14; 14') zumindest abschnittsweise an dessen Außenumfang überlappt.

12. Herzklappenprothese insbesondere nach einem der vorangehenden Ansprüche, bei welcher an einem Klappengestell (2) mehrere Klappensegel (8) befestigt sind, wobei die Klappensegel (8) eine derartige Form aufweisen, dass die im geöffneten Zustand Abschnitte der Umfangsflächen eines Zylinders (L1) definieren.

13. Herzklappenprothese nach Anspruch 14, bei welcher die Klappensegel (8) jeweils eine an das Klappensegel angrenzende Außenkontur aufweisen, welche aus zumindest drei Kreisbögen zusammengefasst sind, wobei zwei äußere Kreisbögen (26) der Außenkontur nach außen und ein oder mehrere zwischen diesen äußeren Kreisbögen gelegene innere Kreisbögen (28, 30) nach innen gekrümmt sind.

## Claims

1. A heart valve prosthesis with a valve frame (2), on which frame several valve leaflets (8) are fastened, as well as with a fastening region which connects axially onto the valve frame (2), for fastening in a blood vessel, **characterised in that** the fastening region comprises a stabilising ring (14; 14', 14", 14‴, 14ʺʺ) which defines a predefined shape and a predefined diameter of the fastening region, the stabilising ring (14; 14', 14", 14‴, 14ʺʺ) comprises commissure supports (18) which project in the axial direction, are fixedly connected to the stabilising ring (14; 14', 14", 14‴, 14ʺʺ) and extend into the commissures (4) formed by the valve frame (2), the stabilising ring (14; 14', 14", 14‴, 14ʺʺ) is provided with radial openings (16; 16'), the stabilising ring (14; 14', 14", 14‴, 14ʺʺ) at least in the radial direction has a lower elastic deformability than the valve frame (2), and is connected to the valve frame (2) in a forcedecoupled manner via a flexible connection so that the forces acting on the fastening region are not transferred to the valve frame (2) and the valve frame (2) is not deformed by the fastening and is not supplied with force.

2. A heart valve prosthesis according to claim 1, **characterised in that** the valve leaflets (8) are manufactured of biological material.

3. A heart valve prosthesis according to claim 1 or 2, **characterised in that** the valve frame (2) is designed in an elastically deformable manner.

4. A heart valve prosthesis according to one of the preceding claims, **characterised in that** the stabilising ring (14; 14', 14", 14‴, 14ʺʺ) is connected to the valve frame (2) via a fabric material (12), wherein the fabric material preferably envelops the valve frame (2) and/or the stabilising ring (14; 14', 14", 14‴, 14ʺʺ).

5. A heart valve prosthesis according to one of the preceding claims, **characterised in that** the stabilising ring (14; 14', 14", 14‴, 14ʺʺ) is designed of metal and in particular of a shape memory alloy.

6. A heart valve prosthesis according to one of the preceding claims, **characterised in that** the stabilising ring (14; 14', 14", 14‴, 14ʺʺ) defines an inner cross section which corresponds to the inner cross section which is delimited by the commissures (4) of the valve frame (2).

7. A heart valve prosthesis according to one of the preceding claims, **characterised in that** the valve frame (2) is elastically deformable in radial direction.

8. A heart valve prosthesis according to one of the preceding claims, **characterised in that** the valve frame (2) in a relaxed condition has a larger outer cross section than the stabilising ring (14; 14', 14", 14‴, 14"").

9. A heart valve prosthesis according to one of the preceding claims, **characterised in that** the stabilising ring (14, 14', 14", 14‴, 14"") has an inner cross section which corresponds to the cross section of the natural blood vessel, in particular the aorta, for which blood vessel the heart valve prosthesis is dimensioned.

10. A heart valve prosthesis according to one of the preceding claims, **characterised in that** the stabilising ring (14') is provided with projections (22) on its outer periphery, said projections being provided for engaging into surrounding tissue and in particular being designed in the form of a thread (22).

11. A heart valve prosthesis according to one of the preceding claims, **characterised by** a flexible suture ring (20) which at least in sections overlaps the stabilising ring (14; 14') at its outer periphery.

12. A heart valve prosthesis according to one of the preceding claims, with which several valve leaflets (8) are fastened on a valve frame (2), wherein the valve leaflets (8) have such a shape that in the opened condition they define sections of the peripheral surfaces of a cylinder (L1).

13. A heart valve prosthesis according to claim 14, with which the valve leaflets (8) each have an outer contour which is adjacent to the valve leaflet and which is composed of at least three arcs, wherein two outer arcs (26) of the outer contour are curved outwards and one or more inner arcs (28, 30) situated between these outer arcs are curved inwards.

## Revendications

1. Prothèse de valve cardiaque avec un bâti pour valves (2) auquel plusieurs feuillets de valve (8) sont fixés, ainsi qu'avec une zone de fixation axialement adjacente au bâti pour valves (2) destinée à la fixation à un vaisseau sanguin,
**caractérisée en ce que**
la zone de fixation comprend un anneau de stabilisation (14 ; 14', 14", 14‴, 14ʺʺ) qui définit une forme donnée et un diamètre donné de la zone de fixation,
l'anneau de stabilisation (14; 14', 14", 14‴, 14ʺʺ) comprend des supports de commissure (18) en excroissance dans la direction axiale et rigidement attachés à l'anneau de stabilisation (14; 14', 14", 14‴, 14ʺʺ), les supports s'étendant dans les commissures (4) formées par le bâti pour valves (2),
l'anneau de stabilisation (14; 14', 14", 14‴, 14ʺʺ) est pourvu d'évidements radiaux (16 ; 16'),
l'anneau de stabilisation (14; 14', 14", 14‴, 14ʺʺ) présente, au moins dans la direction radiale, une capacité de déformation élastique moins grande que le bâti pour valves (2) et est reliée au bâti pour valves (2), sans transmission d'effort, moyennant une liaison flexible, de façon telle que les efforts agissant sur la zone de fixation ne soient pas transmis au bâti pour valves et que le bâti pour valves ne soit pas déformé par la fixation et ne subisse pas d'effort.

2. Prothèse de valve cardiaque selon la revendication 1, **caractérisée en ce que** les feuillets de valve (8) sont fabriqués en matériau biologique.

3. Prothèse de valve cardiaque selon la revendication 1 ou 2, **caractérisée en ce que** le bâti pour valves (2) est formé pour pouvoir changer de forme de manière élastique.

4. Prothèse de valve cardiaque selon l'une des revendications précédentes, **caractérisée en ce que** l'anneau de stabilisation (14; 14', 14", 14‴, 14ʺʺ) est relié au bâti pour valves (2) par un tissu (12), le tissu enveloppant de préférence le bâti pour valves (2) et/ou l'anneau de stabilisation (14 ; 14', 14", 14‴, 14ʺʺ).

5. Prothèse de valve cardiaque selon l'une des revendications précédentes, **caractérisée en ce que** l'anneau de stabilisation (14 ; 14', 14", 14‴, 14ʺʺ) est formé en métal et notamment en un alliage à mémoire de forme.

6. Prothèse de valve cardiaque selon l'une des revendications précédentes **caractérisée en ce que** l'anneau de stabilisation (14; 14', 14", 14‴, 14ʺʺ) définit une section intérieure qui correspond à la section intérieure limitée par les commissures (4) du bâti pour valves (2).

7. Prothèse de valve cardiaque selon l'une des revendications précédentes, **caractérisée en ce que** le bâti pour valves (2) peut être déformé élastiquement en direction radiale.

8. Prothèse de valve cardiaque selon l'une des revendications précédentes, **caractérisée en ce que** le bâti de valves (2) comprend, dans un état détendu, une section extérieure plus grande que l'anneau de stabilisation (14 ; 14', 14", 14‴, 14ʺʺ).

9. Prothèse de valve cardiaque selon l'une des revendications précédentes, **caractérisée en ce que** l'anneau de stabilisation (14; 14', 14", 14‴, 14ʺʺ) comprend une section intérieure qui correspond à la section du vaisseau sanguin naturel, en particulier de l'aorte, pour laquelle la prothèse de valve cardiaque est dimensionnée.

10. Prothèse de valve cardiaque selon l'une des revendications précédentes, **caractérisée en ce que** l'anneau de stabilisation (14') est pourvu, à sa circonférence extérieure, d'excroissances qui sont prévues pour une intervention sur du tissu périphérique et ont notamment la forme d'un filetage (22).

11. Prothèse de valve cardiaque selon l'une des revendications précédentes, **caractérisée par** un anneau de couture (20) flexible qui chevauche l'anneau de stabilisation (14; 14') au moins par section à sa circonférence extérieure.

12. Prothèse de valve cardiaque, notamment selon l'une des revendications précédentes, ayant plusieurs feuillets de valve (8) fixés à un bâti de valves (2), les feuillets de valve ayant une forme telle qu'elles définissent, en l'état ouvert, des sections des surfaces de circonférence d'un cylindre (L1).

13. Prothèse de valve cardiaque selon la revendication 14, les feuillets de valve (8) ayant chacun un contour extérieur adjacent au bâti de valves, lesdites contours étant constitués d'au moins trois arcs de cercle, deux arcs de cercle extérieurs (26) du contour extérieur étant courbés vers l'extérieur et un ou plusieurs arcs de cercle intérieurs (28, 30) disposés entre ces arcs de cercle extérieurs étant courbés vers l'intérieur.
